# EUROPEAN PATENT SPECIFICATION

(11) **EP 1 151 730 B1**
(45) Date of publication and mention of the grant of the patent: **03.01.2007**
(21) Application number: 01304006.8
(22) Date of filing: 02.05.2001
(51) Int. Cl.: A61F 2/06

(54) **Bifurcated stent and stent delivery system**
Abzweigender Stent und Stentanbringungssystem
Stent avec bifurcation et système de mise en place d'un stent

(30) Priority: 02.05.2000 US 562559
(43) Date of publication of application: 07.11.2001
(73) Proprietor: Cordis Corporation, Miami Lakes, Florida 33014 (US)
(72) Inventor: Hojeibane, Hikmat, Princeton, NJ 08540 (US)
(74) Representative: Fisher, Adrian John

(56) References cited:
- EP-A- 0 965 311
- WO-A-96/34580
- WO-A-99/58084
- US-A- 4 733 665
- US-A- 5 102 417
- US-A- 5 697 971
- US-A- 5 738 653
- US-A- 5 938 682

## Description

### TECHNICAL FIELD

The present invention relates to a bifurcated stent endoprosthetic device for implantation within a body vessel, typically a blood vessel, as well as a delivery system for such device.

### BACKGROUND OF THE INVENTION

A type of endoprosthetic device, commonly referred to as a stent, is placed or implanted within a blood vessel for treating occlusions, stenoses, or aneurysms in the blood vessel. These devices are implanted within the vascular system to reinforce collapsing, partially occluded, weakened, or abnormally dilated sections of the blood vessel. Stents also have been successfully implanted in the urinary tract or the bile duct to reinforce those body vessels.

One of the attributes of conventional stents is that they are produced in a straight tubular configuration. The use of such a stent to treat disease at or near a branch or bifurcation of a blood vessel runs the risk of compromising the degree of patency of the primary vessel and/or its branches or bifurcation. This may occur as a result of several problems such as displacing diseased tissue, vessel spasm, dissection with or without intimal flaps, thrombosis, and embolism.

One common procedure for implanting the endoprosthetic or stent is to first open the region of the vessel with a balloon catheter and then place the stent in a position that bridges the weakened portion of the vessel.

In EP-A-0 965 311 there is disclosed a stent comprising a main section having a proximal portion and a distal portion, and a side branch section mating with the main section along a portion of the main section.

In DE-A-19 906 956, which was published after the priority date of this application, there is disclosed a stent of the type set out in claim 1 with the exception that the strut and connector geometries of the distal portion of the main section and the branch section are identical.

### SUMMARY OF THE INVENTION

In accordance with the invention, there is provided a stent as set forth in the accompanying claim 1.

The present invention thus concerns a stent and more particularly a bifurcated stent for insertion into a branching vessel of a subject. A stent constructed in accordance with the invention includes structure that defines a first flow path for fluid to flow through the stent. More particularly, a series of interconnected segments extend axially along and bound this first fluid flow path.

Additional structure, preferably constructed using a second series of interconnected segments, defines a second branching fluid flow path. An interconnection, which is preferably flexible, joins the structure that defines the first and second flow paths. By proper bending of the flexible interconnection the first and second fluid flow paths can be made to conform to a shape of the vessel into which the bifurcated stent is inserted.

The bifurcated stent is constructed from a material that allows the stent to be expanded from an initial shape which can be inserted into a branching vessel to an expanded shape fixed within the branching vessel. A balloon catheter is preferably used to expand the stent by application of outward forces against a series of loops that make up the stent. To deposit the bifurcated stent within the subject vessels, the balloon or balloons are expanded to bring the stent into contact with the inner walls of the vessel and then deflated, leaving the stent in place.

A preferred use for a bifurcated stent constructed in accordance with the present invention is for insertion into a branching blood vessel. A stent constructed in accordance with the present invention is typically intended for use in the coronary vasculature (the right, left common, left anterior descending, and circumflex coronary arteries and their branches) and the peripheral vasculature (branches of the carotid, aorta, femoral, popliteal and other arteries).

A stent constructed in accordance with the invention is also suitable for implantation in other branching vessels of a subject. By way of example the invention has utility for implantation in the gastrointestinal system, the tracheobronchial tree, the biliary system and the genitourinary system.

As described with the present invention, the stent has three segments. Each of the segments is of differing geometry. These differing geometries offer the user an optimal tracking radio-opacity and coverage of the bifurcated portion of the vessels. Further, the geometries provide the ability to accommodate particular variations in the bifurcated side branch of artery. The proximal portion of the main segment of the stent may incorporate a series of angulated struts and flexible omega-shaped connectors. The distal segment of the main portion of the stent is of a variable design. The distal portion of the main leg is designed to have added flexibility without compromising the expansion diameter of the stent. The distal portion of the side branch segment is designed to offer more flexibility to accommodate challenging side branch angles. The main portion of the stent and its distal side branch portion may be connected by a hinge connector firmly joined to both the main branch and the distal branch. This hinge connector is extremely flexible and allows for multiple variations in the vasculature of the patient.

The particular delivery system used with this bifurcated stent encompasses a double-balloon system. The first balloon is placed in the main segment along its entire length. The second balloon is placed within the side branch. Thus, when the stent tracks (in other words, is passed) along the artery, the segmented distal portion of the stent is able to fit nicely into the side branch of the vasculature. The distal side branch segment may have an angled proximal section. This angled proximal section is emplaced within an opening in the main stent segment. The angled proximal section also allows optimal fitting into the main section of the stent. In this fashion, there is full coverage at the bifurcation. (It should be noted that frequently, this bifurcated area is not only difficult to cover fully with a stent, but can more often than not be an area which needs stent expansion due to the build up of plaque at the bifurcation.)

Uniquely, the optional hinge element enables the entire stent to flex one with respect to the other, even after expansion of both stent portions. This modest amount of post-expansion flexing provides for an optimal positioning of the side branch segment with respect to the main branch segment of the stent. Further, the delivery system has two balloons that are attached proximally with the distal tip of the side branch balloon being optionally longer than the distal tip of the main branch balloon.

### DETAILED DESCRIPTION OF THEDRAWINGS

The foregoing invention can be better understood with respect to the appended drawings, in which:
Figure 1 is a perspective view of a bifurcated stent of the present invention;
Figure 2 is a plan view of the bifurcated stent of the present invention;
Figure 3 is a plan view of the stent delivery catheter of the present invention; and
Figures 4A, 4B, 4C, 4D and 4E are schematic views of the stent and delivery system of this invention during use.

### DETAILED DESCRIPTION OF THE DRAWINGS

As mentioned previously, the stent of the present invention has a proximal main segment, which incorporates angled struts, and flexible "omega" shaped connectors. The angled struts allow the stent to house two balloons at its proximal end and to be able to expand proximally to a larger diameter (i.e., larger than a stent with non-angled struts,) without introducing excessive strain on both the stent and the dilating medium, such as the balloon. The most challenging aspects of placing bifurcated stents are coverage by the stent of the carina, prevention of plaque shift at the carina and coverage by the side branch portion of the stent at the proximal end of the side branch opposing the carina. The proximal end of the side branch of the stent of the present invention is designed to address all the concerns mentioned above. The bifurcated stent is designed to fit well into the carina, to prevent "plaque shift" at that location, and to offer optimum coverage of the proximal side branch opposing the carina.

The beveled proximal end of the side branch portion of the bifurcated stent of the present invention also allows for "retraction" of the struts at that end of the stent, as it is being expanded into a side branch that has a larger "take-off' angle (the angle between the main branch artery and its side branch). This retraction of the struts makes the stent less inclined to protrude into the main branch lumen during expansion, thus minimizing the risk of side branch balloon puncturing.

As will be later described, the stent delivery system of the present invention has two balloons that are attached proximally, with the distal tip of the side branch balloon being "longer", that is, extending further distally than the distal tip of the main branch balloon. This feature in the delivery system design offers a few key advantages to the user easy identification of both balloons; facilitation of side branch balloon entry into the side branch; and, more importantly, aiding in properly rotation and orientation of the stent into the bifurcation. Proper stent rotation is needed to align the bifurcated stent's side access slot located in the main branch portion of the stent with the ostium of the side branch.

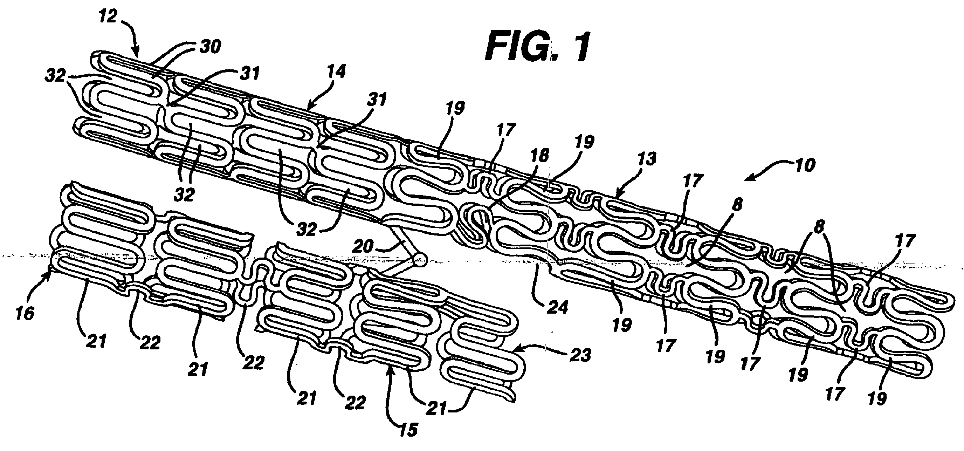

As can be seen from the Figures 1-3 described above, the present bifurcated stent 10 comprises two sections and three distinct portions. There is a main section 12, which has a proximal portion 13 and a distal portion 14. Further, there is described a side branch section 15, which contains a distal portion 16 exclusively. A hinge 20 connects the main section 12 and the side branch section 15. Each of these various sections will be dealt with in order. The stent 10 is generally made from a laser cut tube of surgical grade 316L stainless steel. It is also perceived that other useful materials may be tantalum, plastic or the Naval Ordinance Material known as nitinol, formed from various nickel-titanium compounds. The stent 10 can be manufactured according to well known manufacturing methods, such as those described in Palmaz, US Patent 4,733, 665, as well as other practical tube etching and cutting methods.

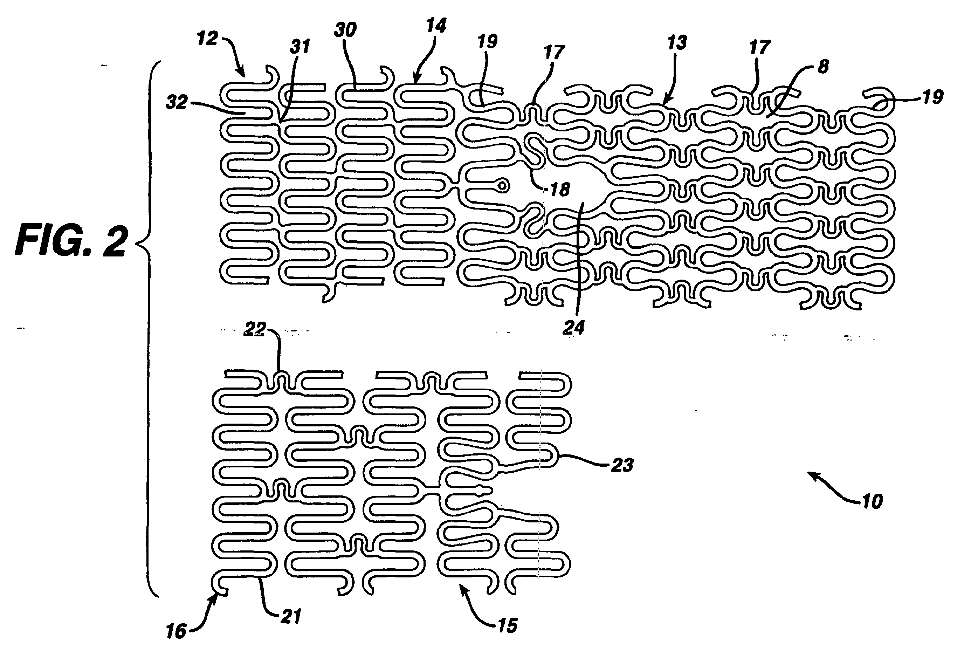

The main section 12 has a proximal portion 13 and a distal portion 14. The distal portion 14 is configured from a series of slotted rings 30 connected by any number of connecting members 31. As best seen in Figure 2, there are contained twelve slots 32 in each ring 30, connected by two connecting members 31. (Of course, to substitute a metallic wire-form bent into slots 31 would be well within the scope of the invention.) Slots 32 and their concomitant connecting members 31 can be much like those described in Palmaz, U.S. Patent No. 5,102,417, owned by a common assignee. The rings 31 form a generally slotted tube portion and lend much structural rigidity to the distal portion 14 of the main section 12 of the bifurcated stent 10 during emplacement.

The proximal portion 13 of the main section 12 of the bifurcated stent 10 is much more flexible in shape. Proximal portion 13 also has a series of generally wavelike sections 19. Omega-like connectors 17 and undulating connectors 18 connect these sections 19. Such omega connector 17 is generally described in U.S. Patent No. 5,938,692. The cells 8 formed from sections 19 and omega connectors 17 are unique in that they are aligned in the same direction along their entire circumferential length. In this fashion, the proximal portion 13 of the main section 12 of the device is intended to be much more flexible than its distal section 14. The omega-like connectors 17 lend a certain greater amount of flexibility than the relatively more rigid slots 31 on the distal portion 14 of the main section 12 of the device.

It should be noted that the distal and proximal sections 14, 13 of the main section 12 of the bifurcated stent 10 need not be expandable to the same diameter. In fact, the branches of a bifurcation tend to be of a different diameter than the proximal section of the bifurcation, such as in a coronary artery. Thus, it is easily envisioned that the proximal section 13 can have a larger inflated diameter than the distal section 14 of main section 12 of the stent 10.

As can be seen from the figures there is furthermore described a side branch section 15. This side branch section 15 is intended to be equally as flexible as the proximal section 13 of the main portions 12. Furthermore, this side branch section 15 is configured from radial struts 21 very much like the configuration of the distal section 14 of the main portion 12. Also, the side branch section 15 is intended to have flexible omega-like connectors 22, such as those found in the proximal portion 13 of the main branch portion 12. Uniquely, the side branch section 15 has a generally angled proximal end 23. This angled proximal end 23 fits neatly into the opening 24 in the proximal portion 13 of the main section 12. Thus, when placed in a bifurcation, the side branch section 15 is placed fully into the bifurcated area of the vasculature.

Significantly, a very flexible hinge 20 connects the main section 12 and the side branch section 15 of the bifurcated stent 10. This hinge 20 is connected to the distal portion 14 of the main section 12 as well as to the proximal end 23 of the side branch section 15. This hinge is made of the same material as the bifurcated stent 10, so as to avoid electrolysis within the body. (Generally, the material used will be a laser cut tube of surgical grade 316L stainless steel. It is also perceived that other useful materials may be tantalum, plastic or the Naval Ordinance Material known as nitinol, formed from various nickel-titanium compounds.) Hinge 20 is flexible throughout its length. In this fashion, the hinge 20 allows one of the sections 13, 15 to flex with respect to the other. Then, upon emplacement at a bifurcation, both the side branch portion 15 and the main branch portion 12 of the bifurcated stent 10 stay firmly emplaced well within their intended positions along the vasculature.

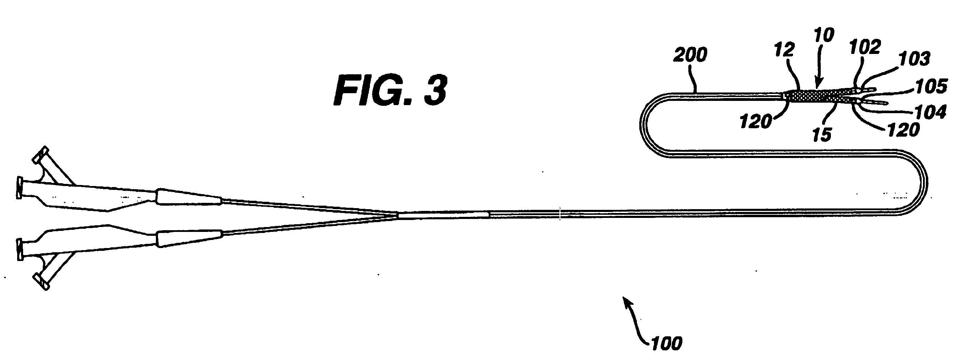

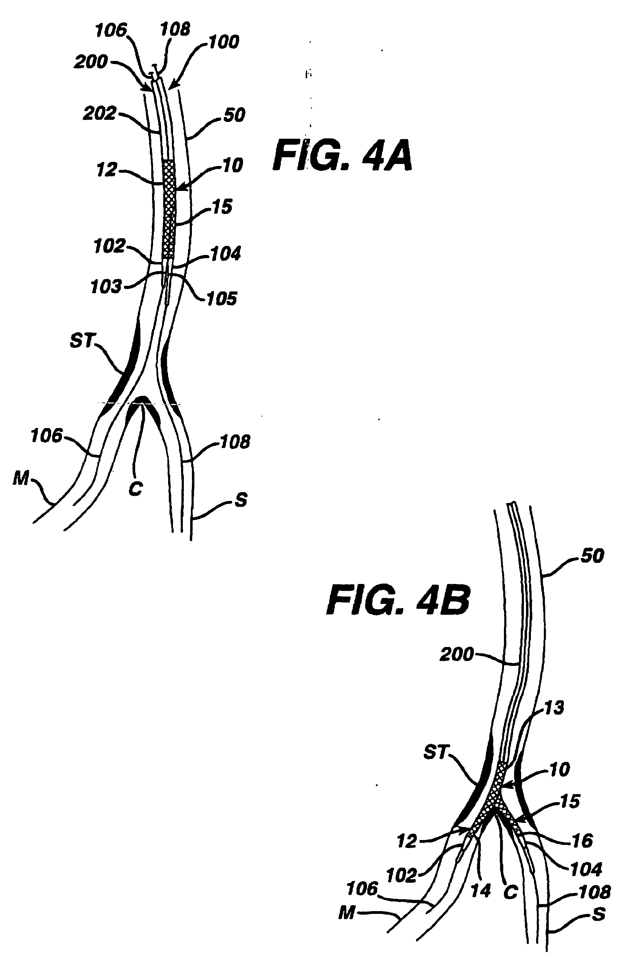

The delivery system associated with this stent 10 is best seen in the accompanying Figures 3, 4A and 4B. Delivery system 100 is generally comprised of two balloons 102, 104 on a two-guidewire system 106, 108. These balloons 102, 104 pass over their respective particular guidewires106, 108. Marker bands 120 are strategically placed along catheter 200.

These balloons 102, 104 similarly carry the bifurcated stent 10 comprising sections 12, 15 with the two sections 12, 15 side-by-side. In this fashion stent 10 is passed entirely along the vasculature. Balloons 102, 104 carrying sections 12, 15 respectively arrive at the bifurcation by passing along guidewires 106, 108. Guide wires 106, 108 are placed within the branches M, S of the bifurcation and the balloons 102, 104 ride over the guidewires 106, 108 to be placed at such points in the bifurcation. The bifurcation is fully covered, the balloons 102, 104 are expanded, the stents sections 12, 15 are put into place and then the balloons 102, 104 are retracted along guidewires 106, 108. Thereafter, guidewires 106, 108 are removed (usually one at a time, like the removal of balloons 102, 104.) Expanded stent 10 is left in place with sections 12, 15 expanded in branches M, S of the bifurcated vasculature. This rather simple positioning, nonetheless, provides for accurate placement of the bifurcated stent 10 of the present device.

Further, it should be noticed that delivery system 100 is somewhat different than other two balloon systems. That is, when laid side by side from the proximal end of the catheter 200, one realizes that distal tip 103 of balloon 102 is closer to proximal end 202 of catheter 200. Or, put another way, distal end 105 of balloon 104 extends about 1 cm further distally than distal end 103. This is intentional. In this fashion, side branch section 15 of stent 10, carried on balloon 104 can be placed at a further distal position than main branch section 12. Because the distal end 105 of the side branch balloon 104 extends further distally than the distal tip 103 of the main branch balloon 102, such placement is facilitated.

Delivery system 100 containing catheter 200 and balloons 102, 104 can be made from well-know methods and materials. For instance, delivery system 100 can be made from the same materials and methods described in Pinchuk, US Patent No. 5,738,653, assigned to a common assignee as the invention herein.

A method of implantation for the bifurcated stent 10 will now be described. As seen in Figure 4A, main trunk 50 of a blood vessel splits into two branches M, S. The two guidewires 106, 108 have been routed through the subject cardiovascular system and into the branches M, S respectively. Techniques for routing guidewires into a subject are well known in the prior art.

Once the two guidewires 106, 108 have been inserted into the position shown in Figure 4A, a bifurcating stent 10 is placed with sections 12, 15 on balloons 102, 104 respectively. Balloons 102, 104 pass over the guidewires 106, 108, to be routed to the branches M, S of vessel 50. A bifurcated stent 10 such as that depicted if Figure 1 is suitable for implantation in the branching vessel 50 depicted in Figure 4A, 4B. The two branching sections 12, 15 of stent 10 are routed separately over the guidewires 106, 108 and guided with the help of a guide catheter into the subject until the Figure 4B position of the stent 10 is achieved at the carina C, in juxtaposition to stenosis ST.

As seen in Figure 4B, in the inserted configuration, the stent 10 passes freely into the subject blood vessel and is ready to be inflated. The stent 10 is typically inserted through a guide catheter (not shown) so that the walls of the stent sections 12, 15 do not engage the inner walls of the blood vessel. To fix the stent 10 in place, balloon 102 inserted main section 12 is inflated to bring the walls of main section 12 including both proximal and distal portions 13, 14 into contact with the blood vessel walls of main branch M. Proximal portion 13 engages the main trunk portion of the blood vessel so that during fabrication of the stent 10 the internal expanded diameter of proximal portion 13 may be chosen to be greater the internal expanded diameter of distal portion 14, as explained above.

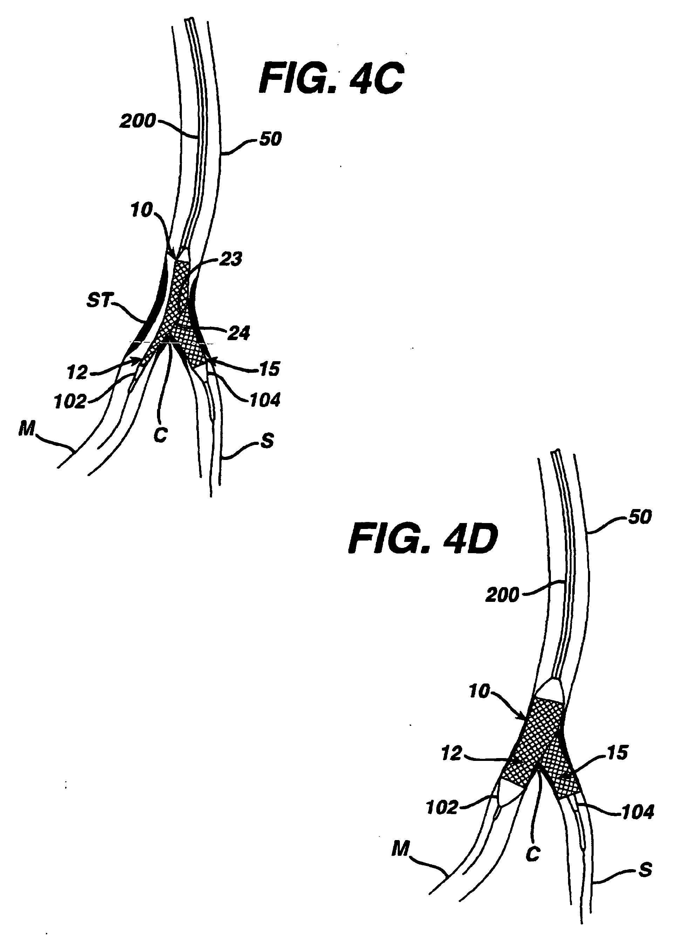

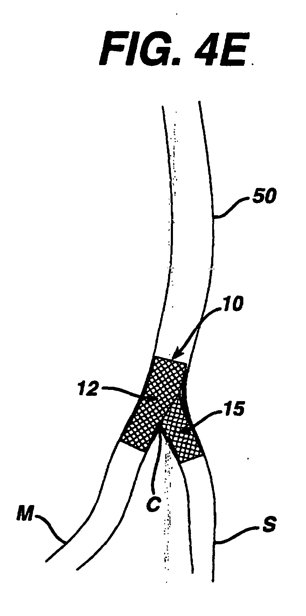

Figure 4C shows smaller dilatation balloon 108 carrying side branch section 15 that is routed through the stent 10 and passes through opening 24 of main section 12 to give access to the side branch S of the bifurcation. When balloon 108 is inflated the side branch section 15 of the stent expands into contact with the side branch S of the vasculature. Proximal portion 23 of side branch section 15 sits firmly within expanded opening 24 of main section 12. Distal end 16 extends just about as far distally as main section 12. Hinge 20 allows for the proper placement of side branch section 15 with respect to main section 12. Ideally, both sections are positioned with hinge 20 lying firmly at the carina C, as can be seen in Figure 4D. Figure 4E shows the fully deployed sections 12, 15 of bifurcated stent 10 supporting the lumens of both the main branch M and side branch S of vessel 50.

A branching or bifurcated stent may be deployed in other ways without resort to a balloon catheter. For example, a bifurcated spring stent may be passed over two guidewires and contained within a guide catheter. Thereafter, the stent could be deployed by passing the guide catheter towards the bifurcation of the vessel, at which time the stent would be ejected into place by advancing a smaller inner catheter through the guide catheter into contact with the stent. The method of deployment of a branching stent will largely depend on the design of the stent and the success of the different deployment techniques for different branching vessel configurations.

The stent 10 may be produced from a variety of materials, either alone or in combination. Useful metals or alloys are stainless steel, titanium, tantalum and nitinol, which can vary in their springiness, malleability, and response to temperature. Also, one can use polymers such as polyurethane, polyether sulfone, polyimide, polycarbonate, and polyethylene, as well as their combinations, which can vary in their ability to be absorbed into tissue or biodegrade. Further one may choose carbon or ceramics. Various surface treatments can be applied to render the stents more biocompatible, such as pyrolytic carbon, and hydrogels. These treatments can also provide for the elution of drugs such as heparin, antiplatelet agents, platelet-derived growth factor, antibiotics, and drugs that help prevent restenosis.

From the above it is to be appreciated that one aspect of the invention is a bifurcated stent which can be expanded from insertion to an in-use configuration. Further, the present invention has been described with a certain degree of particularity. It is intended that the invention include all modifications and alterations from the disclosed embodiments that fall within the scope of the appended claims, as well as all equivalents to which the applicant is entitled.

## Claims

1. A stent (10) comprising:
a main section (12) having a distal portion (14) and a proximal portion (13); and
a side branch section (15), said side branch section (15) mating with said main section (12) along a portion of said main section; **characterized in that** each of said distal portion (14), said proximal portion (13) and said side branch section (15) comprise struts (19; 21; 30) connected by connectors (17; 22; 31); and
wherein each of said distal portion (14), said proximal portion (13) and said side branch section (15) have different strut and connector geometries.

2. The stent of claim 1, wherein said main section (12) and said side branch section (15) are connected by a hinge (20).

3. The stent of claim 1 or 2, wherein said stent (10) is balloon expandable.

4. The stent of claim 1 or 2, wherein the stent (10) is self-expanding.

5. The stent of claim 1 or 2 wherein the side branch section (15) has a beveled proximal end (23).

6. The stent of claim 1 or 2 wherein the side branch section (15) contains a plurality of cells connected by flexible connectors (22).

7. The stent of claim 1 or 2 wherein said proximal portion (13) is relatively rigid and said distal portion (14) is relatively flexible with respect to one another.

8. In combination:
a stent (10) in accordance with any preceding claim; and
a stent delivery system (100) comprising a pair of balloons (102, 104), wherein a first of said balloons (102) is configured to carry said main section (12) and the second of said balloons (104) is configured to carry said side branch section (15).

9. The combination of claim 8 wherein said first of said balloons (102) extends further distally than the second of said balloons (104).

10. The combination of claim 8 or claim 9, wherein said balloons (102, 104) have different lengths.

## Patentansprüche

1. Stent (10), der folgendes umfaßt:
einen Hauptabschnitt (12) mit einem distalen Teil (14) und einem proximalen Teil (13); und
einen Seitenabzweigungsabschnitt (15), wobei der Seitenabzweigungsabschnitt (15) mit dem Hauptabschnitt (12) entlang eines Teils des Hauptabschnitts verbunden ist; **dadurch gekennzeichnet, daß** der distale Teil (14), der proximale Teil (13) und der Seitenabzweigungsabschnitt (15) Streben (19; 21; 30) aufweisen, die durch Verbinder (17; 22; 31) verbunden sind; und
wobei der distale Teil (14), der proximale Teil (13) und der Seitenabzweigungsabschnitt (15) unterschiedliche Streben- und Verbindergeometrien aufweisen.

2. Stent nach Anspruch 1, bei welchem der Hauptabschnitt (12) und der Seitenabzweigungsabschnitt (15) durch ein Gelenk (20) verbunden sind.

3. Stent nach Anspruch 1 oder 2, wobei der Stent (10) durch einen Ballon ausdehnbar ist.

4. Stent nach Anspruch 1 oder 2, wobei der Stent (10) selbstausdehnend ist.

5. Stent nach Anspruch 1 oder 2, bei dem der Seitenabzweigungsabschnitt (15) ein abgeschrägtes proximales Ende (23) aufweist.

6. Stent nach Anspruch 1 oder 2, bei dem der Seitenabzweigungsabschnitt (15) mehrere Zellen aufweist, die durch flexible Verbinder (22) verbunden sind.

7. Stent nach Anspruch 1 oder 2, bei dem der proximale Teil (13) vergleichsweise steif ist und der distale Teil (14) gegenüber dem Anderen vergleichsweise flexibel ist.

8. In Verbindung:
eine Stent (10) gemäß einem der vorhergehenden Ansprüche; und
ein Stentzuführungssystem (100), das ein Paar Ballons (102, 104) aufweist, wobei ein erster der Ballons (102) dafür eingerichtet ist, den Hauptabschnitt (12) zu stützen, und der zweite der Ballons (104) dafür eingerichtet ist, den Seitenabzweigungsabschnitt (15) zu stützen.

9. Kombination nach Anspruch 8, wobei der erste der Ballons (102) sich weiter distal erstreckt als der zweite der Ballons (104).

10. Kombination nach Anspruch 8 oder 9, wobei die Ballons (102, 104) unterschiedliche Längen aufweisen.

## Revendications

1. Stent (10) comprenant :
- une section principale (12) possédant une partie distale (14) et une partie proximale (13) ; et
- une section de bifurcation latérale (15), laquelle section de bifurcation latérale (15) correspond à ladite section principale (12) le long d'une partie de cette dernière ;
**caractérisé en ce que** chacune desdites partie distale (14), partie proximale (13) et section de bifurcation latérale (15) comprend des entretoises (19 ; 21 ; 30) connectées par des connecteurs (17 ; 22 ; 31) ; et
dans lequel chacune desdites partie distale (149, partie proximale (13) et section de bifurcation latérale (15) possède des géométries d'entretoises et de connecteurs différentes.

2. Stent selon la revendication 1, dans lequel ladite section principale (12) et ladite section de bifurcation latérale (15) sont connectées par une charnière (20) .

3. Stent selon la revendication 1 ou 2, lequel stent (10) peut être déployé à l'aide d'un ballonnet.

4. Stent selon la revendication 1 ou 2, lequel stent (10) se déploie automatiquement.

5. Stent selon la revendication 1 ou 2, dans lequel ladite section de bifurcation latérale (15) possède une extrémité proximale biseautée (23).

6. Stent selon la revendication 1 ou 2, dans lequel ladite section de bifurcation latérale (15) comprend plusieurs cellules connectées par des connecteurs flexibles (22).

7. Stent selon la revendication 1 ou 2, dans lequel ladite partie proximale (13) est relativement rigide et ladite partie distale (14) est relativement flexible, l'une par rapport à l'autre.

8. Combinaison comprenant :
- un stent (10) selon l'une quelconque des revendications précédentes ; et
- un système d'acheminement de stent (1.00) comprenant deux ballonnets (102, 104), dans lequel le premier ballonnet (102) est configuré pour supporter ladite section principale (12), tandis que le second ballonnet (104) est configuré pour supporter ladite section de bifurcation latérale (15).

9. Combinaison selon la revendication 8, dans laquelle ledit premier ballonnet 102 s'étend distalement plus en avant que le second ballonnet (104).

10. Combinaison selon la revendication 8 ou la revendication 9, dans laquelle lesdits ballonnets (102, 104) ont des longueurs différentes.
